# EUROPEAN PATENT SPECIFICATION

(11) **EP 3 124 066 B1**
(45) Date of publication and mention of the grant of the patent: **26.09.2018**
(21) Application number: 15768110.7
(22) Date of filing: 29.01.2015
(51) Int. Cl.: A61M 5/145, A61M 5/168, A61M 5/142

(54) **LIQUID-DRUG ADMINISTRATION DEVICE**
FLÜSSIGARZNEIMITTELVERABREICHUNGSVORRICHTUNG
DISPOSITIF D'ADMINISTRATION DE MÉDICAMENT LIQUIDE

(30) Priority: 26.03.2014 JP 2014062940
(43) Date of publication of application: 01.02.2017
(73) Proprietor: Terumo Kabushiki Kaisha, Tokyo 151-0072 (JP)
(72) Inventor: OYAMA, Takatoshi, Kurokawa-gun Miyagi 981-3626 (JP); KONDO, Akira, Ashigarakami-gun Kanagawa 259-0151 (JP)
(74) Representative: Casalonga
(86) International application number: PCT/JP2015/052467
(87) International publication number: WO 2015/146276

(56) References cited:
- WO-A1-2013/150025
- JP-A- H09 250 934
- JP-A- 2001 029 462
- JP-A- 2007 511 252
- JP-A- 2008 264 140
- US-A1- 2004 133 166
- US-A1- 2005 267 439
- US-A1- 2010 292 651

## Description

### Technical Field

The present invention relates to a liquid-drug administration device, in particular, to a handheld liquid-drug administration device for continuously administrating liquid-drug, such as an insulin pump. In particular, the present invention relates to a liquid-drug administration device according to the preamble of claim 1, auch as it is for example known from WO-A-2013150025.

### Background Art

In recent years, cure by continuously administrating liquid-drug into the body of a patient by, for example, subcutaneous injection or intravenous injection, has been performed. For example, a slight amount of insulin is continuously administrated into the body of a patient to cure diabetes. In such a cure, a handheld liquid-drug administration device (called an insulin pump), which can be secured on the body or clothes of a patient to be carried, has been used to administrate liquid-drug (insulin) into a patient throughout the day.

As such a handheld liquid-drug administration device, a syringe pump device including a syringe storing liquid-drug and a plunger which is driven in the syringe is proposed. Such a liquid-drug administration device is provided with an occlusion-sensor that detects occlusion in a liquid-drug passage occurred by some cause. As an occlusion-sensor, for example, an encoder that controls rotation of a motor for driving a plunger and a configuration for detecting occlusion by using a pressure sensor including a diaphragm provided at where liquid-drug is discharged are disclosed (see Patent Literature 1 listed below).

### Citation List

### Patent Literature

Patent Literature 1: JP 2013-537844 A (Figs. 39 to 41, Fig 93, and related parts)

### Summary of Invention

### Technical Problem

Detection of occlusion with high accuracy using such an occlusion-sensor is however difficult. An object of the present invention is to provide a liquid-drug administration device that can detect occlusion with high accuracy.

### Solution to Problem

To achieve the aforementioned object, the present invention provides a liquid-drug administration device according to claim 1. The dependent claims relate to advantageous embodiments.

The occlusion detector configured as described above serves as an encoder to detect overlapping of slits of the fixed slit disk and the slit disk. The rise in the back pressure on the output shaft can thus be detected more correctly than an encoder using only a single slit disk.

### Advantageous Effects of Invention

The liquid-drug administration device according to the present invention as described above detects the rise in the back pressure on the output shaft with higher correctness to enable occlusion detection with high accuracy.

### Brief Description of Drawings

Fig. 1 is a schematic plan view illustrating a general configuration of a liquid-drug administration device.
Fig. 2 is an exploded perspective view of an essential portion of the liquid-drug administration device.
Figs. 3A and 3B are plan views for explaining a configuration of an occlusion detector.
Figs. 4A and 4B are exploded perspective views for explaining assembly of the occlusion detector.
Figs. 5A, 5B, and 5C are figures for explaining a signal detected by an optical sensor of the occlusion detector.
Figs. 6A and 6B are a first group of figures for explaining occlusion detection in the liquid-drug administration device.
Figs. 7A and 7B are a second group of figures for explaining occlusion detection in the liquid-drug administration device.
Figs. 8A and 8B are a third group of figures for explaining occlusion detection in the liquid-drug administration device.
Fig. 9 is a perspective view illustrating a second example of an actuator provided in the liquid-drug administration device.
Fig. 10 is a plan view illustrating a second example of an output shaft and a plunger provided in the liquid-drug administration device.

### Description of Embodiments

An embodiment of a liquid-drug administration device according to the present invention will now be described in detail with reference to the drawings. The liquid-drug administration device according to the present invention is widely used for a handheld liquid-drug administration device for continuously administrating liquid-drug into the body of a patient, such as an insulin patch pump, an insulin tube pump, and other handheld liquid-drug administration devices.

### Configuration of Liquid-Drug Administration Device

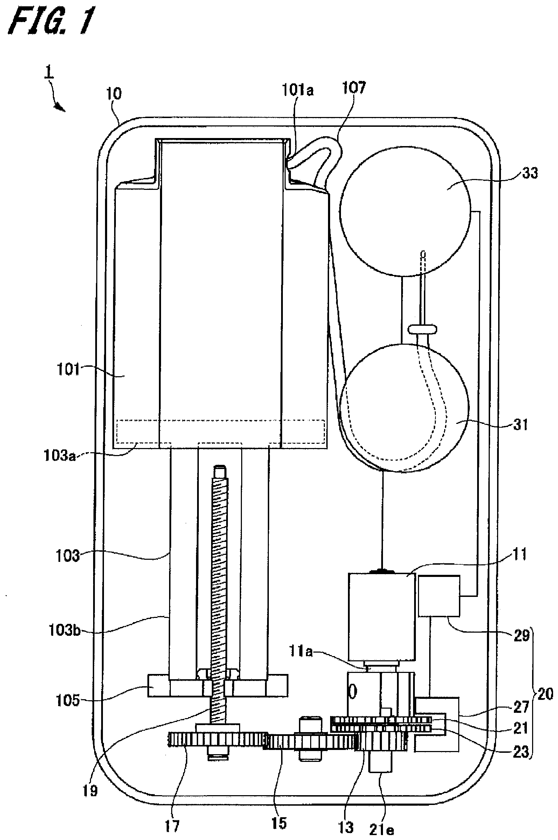

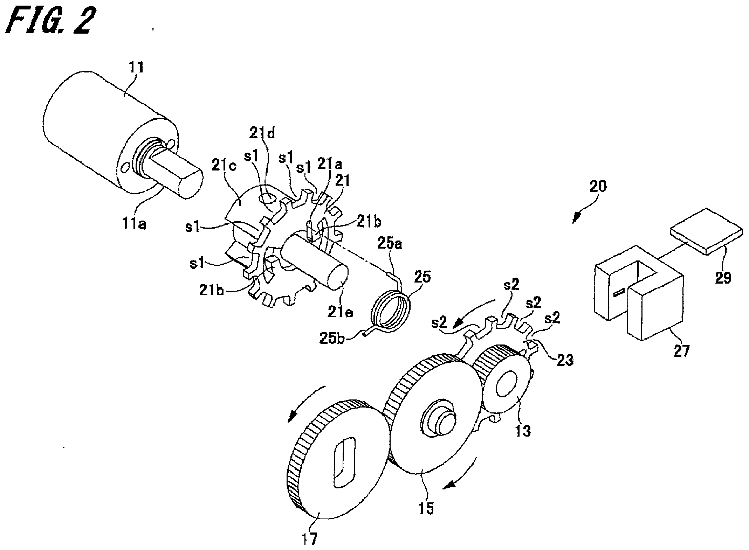

Fig. 1 is a schematic plan view illustrating a general configuration of a liquid-drug administration device 1 in which the present invention is used. Fig. 2 is an exploded perspective view of an essential portion of the liquid-drug administration device 1 in which the present invention is used. The liquid-drug administration device 1 illustrated in the drawings is used as an insulin pump, for example, and configured as described below.

The liquid-drug administration device 1 includes, inside a case 10, a motor 11 as an example of an actuator, a plurality of stages of gears 13, 15, and 17 that are rotated by the motor 11, and an advance screw 19 as an example of an output shaft provided on the rotation axis of the final-stage gear 17 among the gears 13, 15, and 17. In this configuration, an occlusion detector 20, which is unique to the liquid-drug administration device 1 according to the embodiment, is provided between the motor 11 and the advance screw 19.

The liquid-drug administration device 1 described above may include a battery box 31 that houses a battery for driving the motor 11, and an alarm unit 33 connected to the occlusion detector 20. The liquid-drug administration device 1 includes a syringe 101 as a liquid-drug container, a plunger 103 inserted in the syringe 101, and a nut 105 fixed to the plunger 103 and screwed on the advance screw 19. The syringe 101, the plunger 103, and the advance screw 19 are provided along the axial direction of the advance screw 19. Each of the components described above will now be described.

### Motor 11 (Actuator)

The motor 11 is driven by a battery and includes a rotation shaft 11a that is rotated. The motor 11 is, for example, a stepping motor driven by pulses.

### Gears 13, 15, 17

The gears 13, 15, and 17 are meshed to each other to be rotated in conjunction with the driven motor 11. The gear 13 is a first-stage gear 13 provided on the rotation shaft 11a of the motor 11. The gear 15 is an intermediate gear 15 provided to mesh with the first-stage gear 13 to reduce speed. The gear 17 is a final-stage gear 17 provided to mesh with the intermediate gear 15. The final-stage gear 17 is in meshed relationship with the first-stage gear 13 via the intermediate gear 15. The advance screw 19 is fixed on the rotation axis of the final-stage gear 17.

The gears 13, 15, and 17 are configured so as the rotation of the motor 11 to be transmitted to the final-stage gear 17 via the first-stage gear 13 and the intermediate gear 15. The advance screw 19 is fixed to the final-stage gear 17 so that the final-stage gear 17 rotates in synchronization with the rotation of the advance screw 19 and thereby the intermediate gear 15 and the first-stage gear 13, which are in meshed relationship with the final-stage gear 17, rotate in synchronization with the advance screw 19.

Such gears 13, 15, and 17 may be, for example, spur gears illustrated in the drawings. The intermediate gear 15 may be provided as required and may be provided as a plurality of meshed gears.

### Advance Screw 19 (Output Shaft)

The advance screw 19 is fixed upright on the final-stage gear 17 to be on the rotation axis of the final-stage gear 17. The advance screw 19 slides the plunger 103 in the syringe 101 described below. The advance screw 19 has a thread having a pitch corresponding to the advancing speed of the plunger 103 and an effective length corresponding to the moving range of the plunger 103. For example, the plunger 103 moves away from the final-stage gear 17 by the left rotation of the final-stage gear 17. The plunger 103 moves toward the final-stage gear 17 by the right rotation of the final-stage gear 17.

### Occlusion Detector 20

The occlusion detector 20 detects occlusion in a flow passage for liquid-drug supplied from the syringe 101 described below. For example, the occlusion detector 20 is configured as an encoder disposed between the motor 11 and the first-stage gear 13. The occlusion detector 20 includes a fixed slit disk 21, a slit disk 23, and a torsion spring 25 (see Fig. 2). The fixed slit disk 21 is positioned closer to the motor 11 than the slit disk 23, and the torsion spring 25 is disposed between the fixed slit disk 21 and the slit disk 23. The occlusion detector 20 includes an optical sensor 27 and a processing unit 29 connected to the optical sensor 27.

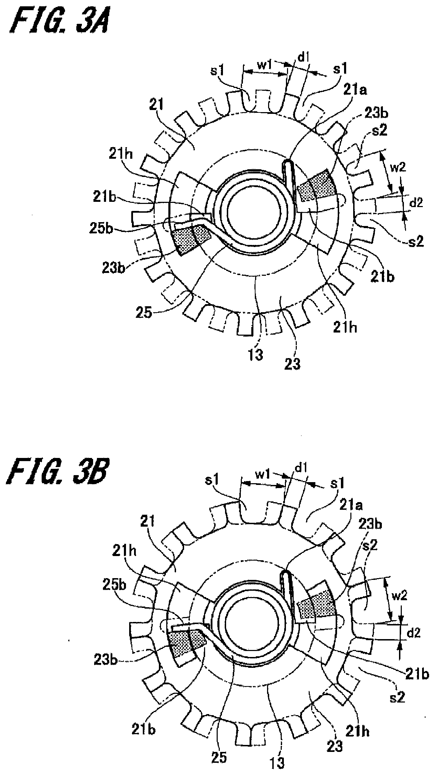

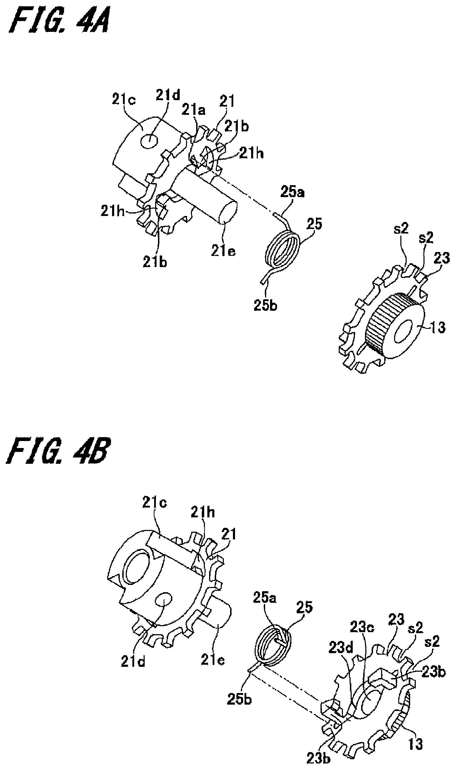

The detail on the configuration of the occlusion detector 20 will now be described below with reference to Figs. 1 and 2 as well as Figs. 3A to 4B. Figs. 3A and 3B are plan views for explaining the configuration of the occlusion detector 20, where the slit disk 23, the fixed slit disk 21, and the torsion spring 25 are viewed from the first-stage gear 13. In Figs. 3A and 3B, the first-stage gear 13 and the slit disk 23 are illustrated in virtual lines (two-dot chain lines). Figs. 4A and 4B are exploded perspective views for explaining assembly of the occlusion detector 20. Figs. 4A and 4B illustrate the occlusion detector 20 viewed from the opposite side.

### Fixed Slit Disk 21

The fixed slit disk 21 is fixed to the rotation shaft 11a of the motor 11 by the center of the disk face to rotate in synchronization with the rotation of the motor 11. The fixed slit disk 21 includes a slit s1 in its disk face. The rim of the fixed slit disk 21 is cut out to create a plurality of slits s1. The slits s1 each has a predetermined opening width w1 in a circumferential direction. Between the slits s1 along the rim of the fixed slit disk 21, for example, a predetermined distance d1 is provided.

For example, 13 slits s1 are disposed on the rim of the fixed slit disk 21 with the even distance d1 therebetween. The opening width w1, in the circumferential direction, of the slit s1 is three times the distance d1 provided between the slit s1 and the slit s1 (w1 = d1 × 3) (see Fig. 3).

The fixed slit disk 21 includes a positioning hole 21a for positioning and fixing an end 25a of the torsion spring 25 on the disk face opposing the slit disk 23. The end 25a of the torsion spring 25, namely one of two arms of the torsion spring 25, is inserted in the positioning hole 21a along a direction approximately vertical to the disk face of the fixed slit disk 21. The inserted end 25a is restricted from moving in the circumferential direction of the fixed slit disk 21.

The fixed slit disk 21 includes a plurality of tabs 21b for assembling the slit disk 23. A bore is created in the disk face of the fixed slit disk 21 to form two tabs 21b projecting in the circumferential direction but kept within the disk face of the fixed slit disk 21. The two tabs 21b project in the same direction and are disposed at locations, for example, separated from each other by 180 degrees about the center of the fixed slit disk 21. The bore provided in the fixed slit disk 21 to create the tabs 21b in the fixed slit disk 21 also serves as an engage hole 21h into which a fitting portion 23b of the slit disk 23, which will be described below, is inserted.

A fixing portion 21c in which the rotation shaft 11a of the motor 11 fits is provided on the disk face of the fixed slit disk 21 opposing the motor 11 so as the disk face of the fixed slit disk 21 to be vertical to the rotation shaft 11a of the motor 11. The fixing portion 21c is provided with a nut 21d to secure the rotation shaft 11a fitted in the fixing portion 21c.

A shaft portion 21e that coaxially positions and supports the torsion spring 25 and the slit disk 23 on the rotation shaft 11a of the motor 11 is provided upright on the fixed slit disk 21 in the center of the disk face opposing the slit disk 23.

### Slit Disk 23

The slit disk 23 is concentrically fixed to the first-stage gear 13. The slit disk 23 may be integrally formed with the first-stage gear 13. The slit disk 23 is provided so as to rotate in synchronization with the rotation of the advance screw 19.

A plurality of slits s2 is provided in the disk face of the slit disk 23. The slits s2 overlap the slits s1 of the fixed slit disk 21 when the slit disk 23 coaxially overlaps the fixed slit disk 21. For example, the outer circumferential shape of the slit disk 23 is identical to the outer circumferential shape of the fixed slit disk 21. Evenly spaced thirteen slits s2 are provided on the rim of the slit disk 23. The opening width w2 (= w1) in the circumferential direction of the slit s2 is three times the distance d2 (= d1) provided between the slit s2 and the slit s2 (w2 = d2 × 3) (see Fig. 3).

The slit disk 23 is coaxially and overlappingly assembled to the fixed slit disk 21. The slit disk 23 is provided with a fitting portion 23b on the disk face opposing the fixed slit disk 21. The fitting portion 23b is inserted in the engage hole 21h of the fixed slit disk 21 to engage with the tab 21b of the fixed slit disk 21. The fitting portion 23b projects from the disk face of the slit disk 23 and has a shape allowing the tab 21b of the fixed slit disk 21 to slide to engage with the fitting portion 23b. Two fitting portions 23b corresponding to the tabs 21b of the fixed slit disk 21 are provided. The tab 21b of the fixed slit disk 21 engages with each of the fitting portions 23b of the slit disk 23 to assemble the slit disk 23 to the fixed slit disk 21.

With the fixed slit disk 21 and the slit disk 23 assemble together, the fixed slit disk 21 and the slit disk 23 can rotate relative to each other within a predetermined angular range with the disk faces opposing each other.

One of the two fitting portions 23b provided on the slit disk 23 serves as a securing member for securing the other end 25b of the torsion spring 25, that is, the other one of the two arms, on the slit disk 23.

A shaft hole 23c which the shaft portion 21e of the fixed slit disk 21 penetrates is provided in the center of the slit disk 23. The shaft hole 23c may penetrate the center of the first-stage gear 13. The slit disk 23 is provided with a housing portion 23d having a form of a recess to house the torsion spring 25. The housing portion 23d is provided in the disk face opposing the fixed slit disk 21 and includes therein the shaft hole 23c.

### Torsion Spring 25

The torsion spring 25 connects together the fixed slit disk 21 fixed to the motor 11 and the slit disk 23 fixed to the first-stage gear 13 in an overlapping manner. The torsion spring 25 is provided as an elastic member that causes a delay in the rotation of the slit disk 23 with respect to the rotation of the fixed slit disk 21 in response to the back pressure on the first-stage gear 13 and the slit disk 23 fixed to the first-stage gear 13. In this manner, the torsion spring 25 together with the fixed slit disk 21 and the slit disk 23 constitute a back torque limiter.

A wire wound in a form of a coil constitutes the torsion spring 25. The end 25a of the torsion spring 25 is bent to a direction vertical to the flexing direction of the torsion spring 25. The other end 25b of the torsion spring 25 extends to the outer side of the winding direction of the coil.

The torsion spring 25 is disposed with the wound portion fitted around the shaft portion 21e of the fixed slit disk 21 and the end 25a inserted in the positioning hole 21a of the fixed slit disk 21.

With the end 25a fixed, a load is applied to the torsion spring 25 in the flexing direction by one of the fitting portions 23b projecting from the slit disk 23. Keeping this loading, the fitting portion 23b of the slit disk 23 is inserted in the engage hole 21h of the fixed slit disk 21. The resilience of the torsion spring 25 causes the tab 21b of the fixed slit disk 21 to engage with the fitting portions 23b of the slit disk 23 to assemble together the fixed slit disk 21 and the slit disk 23.

In the initial state of the assembled fixed slit disk 21 and slit disk 23, for example, the slits s1 are positioned to be shifted from the slits s2 by half a pitch. In this state, the slits s1 and the slits s2 overlap each other in a manner that the protrusion provided between the slits s2 of the slit disk 23 is positioned in the middle of the slit s1 of the fixed slit disk 21 (as illustrated in Fig. 3A).

Under a normal condition where the slit disk 23 assembled as described above receives a back pressure within a normal range, the torsion spring 25 keeps the initial state described above and thus has a coefficient of spring sufficient enough to rotate the slit disk 23 in synchronization with the fixed slit disk 21.

As the back pressure on the slit disk 23 rises, the coefficient of spring flexes by a flexing degree corresponding to the rise in the back pressure to cause a delay in rotation of the slit disk 23 with respect to the rotation of the fixed slit disk 21 by a predetermined degree. The coefficient of spring is set considering the degree of occlusion in a flow passage for liquid-drug squeezed out from a syringe 101, which will be described below, and the degree of rise in the back pressure corresponding to the degree of occlusion. It is important that the coefficient of spring of the torsion spring 25 should be set so as the fitting portion 23b of the slit disk 23 not to disengage from the tab 21b of the fixed slit disk 21 at the estimated maximum back pressure during an operation of the liquid-drug administration device 1.

### Optical Sensor 27

The optical sensor 27 (see Fig. 1 and Fig. 2) detects overlapping of the slit s1 of the fixed slit disk 21 and the slit s2 of the slit disk 23. For example, a transmissive optical sensor 27 including a light-emitting element and a light-receiving element opposing each other is used. The
light-emitting element is a light emitting diode (LED), for example, and the light-receiving element is a photo diode (PD), for example. The light-emitting element may be one of a pulse-transmitting type. In the transmissive optical sensor 27, the light emitted from the light-emitting element and received by the light-receiving element (hereinafter referred to as detection light) passes a measuring point p for light detection. Thus, the transmissive optical sensor 27 is fixed in the case 10 so as the rim of the fixed slit disk 21 and the rim of the slit disk 23 are disposed between the light-emitting element and the light-receiving element.

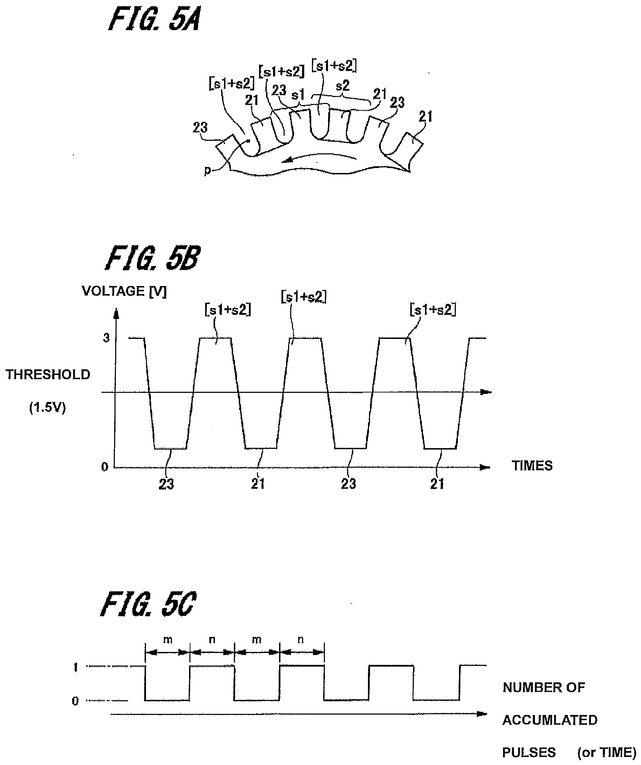

Figs. 5A, 5B, and 5C are figures for explaining detection of a signal by the optical sensor 27. As illustrated in Fig. 5A, the measuring point p of the optical sensor 40 is fixed at a predetermined point in a region where the rim of the fixed slit disk 21 and the rim of the slit disk 23 are disposed between the light-emitting element and the light-receiving element. When the fixed slit disk 21 and the slit disk 23 are rotated, a detection light emitted from the light-emitting element is received by the light-receiving element only while the region where the slit s1 overlaps the slit s2 [s1 + s2] passes the measuring point p.

As illustrated in Fig. 5B, the detection light received by the light-receiving element is photoelectrically converted and output as a voltage signal. A state in which the region where the slit s1 overlaps the slit s2 [s1 + s2] passes the measuring point p and a state in which the fixed slit disk 21 or the slit disk 23 blocks the detection light are detected by a voltage difference. By setting a threshold for the voltage detected by the light-receiving element (for example, 1.5 V), a state in which the overlapping region [s1 + s2] passing the measuring point p is detected by detecting the voltage equal to or higher than the threshold.

As illustrated in Fig. 5C, detection of the voltage equal to or higher than the threshold by the light-receiving element can digitally be distinguished from detection of other voltages by 0 and 1. When a light-emitting element is of a pulse-transmitting type, detection signals "1" are continuously counted a predetermined times in an overlapping region [s1 + s2] along the horizontal axis representing the number of accumulated pulses, and for other regions, detection signals "0" are continuously counted a predetermined times.

In the initial state described above, the slits s1 overlap the slits s2 in a manner that the protrusion provided between the slits s2 of the slit disk 23 is positioned in the middle of the slit s1 of the fixed slit disk 21. Thus, the period in which the detection signals "1" are continuously counted n times in the overlapping region [s1 + s2] and the period in which the detection signals "0" are continuously counted m times are alternately detected by detection signals.

Detection of occlusion based on the detection signals described above is performed by a processing unit 29 which will now be described.

The optical sensor 27 is not limited to the transmissive optical sensor described above. A reflective optical sensor may be used depending on the internal layout of the case 10. When a reflective optical sensor is used, a portion of the detection light, emitted from the light-emitting element, that has reflected on the fixed slit disk 21 or the slit disk 23 is received by the light-receiving element. Thus, when the light-receiving element receives no detection light, it is detected that the region where the slit s1 overlaps the slit s2 is passing the point where the optical sensor is positioned (measuring point).

### Processing Unit 29

The processing unit 29 is a signal processing portion that determines whether occlusion has occurred in a flow passage for liquid-drug supplied, by the driving of the advance screw 19, from the syringe 101 based on a signal obtained by the optical sensor 27. When the processing unit 29 determines that occlusion has occurred in the flow passage, the processing unit 29 sends a signal to an alarm unit 33 to set forth an alarm. The algorithm used in the processing unit 29 to determine whether occlusion has occurred in a flow passage will be described in detail in the description on a method for occlusion detection. The alarm unit 33 that sets forth an alarm by, for example, vibration or sound, or those accompanied by light is used.

In the processing unit 29, a signal obtained by the optical sensor 27 of the occlusion detector 20 is fed back into a signal for driving the motor 11 to control the driving of the motor 11.

### Syringe 101 (liquid-drug container)

The syringe 101 is a sleeve-shaped liquid-drug storage that has a closed bottom end and an opened bottom end. The syringe 101 has a straight sleeve shape allowing the plunger 103 to slide therein in the extending direction of the cylinder. To prevent the plunger 103 from rotating when sliding in the syringe 101, the bottom of the sleeve preferably has a flat shape, such as an ellipse. The syringe 101 has a length in the extending direction of the sleeve which is approximately the same as the length of the advance screw 19.

The syringe 101 has a supply port 101a for liquid-drug at its occlusion bottom end. For example, the proximal end of a tube 107 having a needle at its distal end is connected to the supply port 101a.

The tube 107 is fixed in the case 10 with the distal end of the needle intruding into the liquid-drug outflow port of the case 10, which outflow port being omitted in the drawing. If the liquid-drug administration device 1 is of a patch type, a cradle is prepared to place thereon the case 10 and the distal end of the tube 107 communicates with a piercing needle fixed to the cradle. If the liquid-drug administration device 1 is of a tube type, the tube having a piercing needle fixed to its distal end is connected to the liquid-drug outflow port of the case 10.

### Plunger 103

The plunger 103 is slidably inserted in the syringe 101, keeping liquid-tightness, to adjust the volume inside the syringe 101 as required. The plunger 103 includes, for example, a head 103a which is inserted in the syringe 101, and a shaft 103b provided vertically upright on the head 103a. The shaft 103b has approximately the same length as the advance screw 19.

### Nut 105

The nut 105 is fixed to the plunger 103 and screwed on the advance screw 19. The nut 105 is fixed to the rear end of the shaft 103b of the plunger 103, that is, to the opposite side of the head 103a.

With this configuration, rotation of the advance screw 19 fixed to the final-stage gear 17 causes the nut 105 screwed on the advance screw 19 to move in the extending direction of the advance screw 19. The plunger 103 fixed to the nut 105 thereby slides in the extending direction of the advance screw 19 in the syringe 101 to increase or decrease the volume inside the syringe 101.

The nut 105 described above may be a half-nut detachably provided on the advance screw 19. In such a case, the syringe 101 and the plunger 103 can be detached from the main body of the liquid-drug administration device 1 provided with the motor 11 and the occlusion detector 20 via the nut 105. In this case, the liquid-drug administration device 1 includes the motor 11, the gears 13, 15, and 17, the advance screw 19, the occlusion detector 20, and the alarm unit 33, which are provided in the case 10 as reusable parts that can repetitively be used. Meanwhile, the syringe 101, the plunger 103, the tube 107, and the battery box 31, which are exposed to liquid-drug, are detachably provided in a separated case as disposable parts.

The nut 105 is not necessarily provided as a half-nut to constitute the liquid-drug administration device 1 composed of reusable parts and disposable parts. For example, the nut 105 may be a full-nut that can be detached from the shaft 103b with the advance screw 19 screwed in the full-nut.

If the liquid-drug administration device 1 needs not be composed of reusable parts and disposable parts, the nut 105 is not necessarily be a half-nut but may be a normal ring nut. In such a case, the plunger 103 may be composed only of the head 103a provided as a nut through which center the advance screw 19 penetrates.

### Operation of Liquid-Drug Administration Device

The liquid-drug administration device 1 configured as described above is operated as follows.

When administrating liquid-drug, the motor 11 is driven in a predetermined direction to rotate the fixed slit disk 21 fixed to the rotation shaft 11a of the motor 11 in a predetermined direction in synchronization with the motor 11. With this rotation, the slit disk 23 assembled to the fixed slit disk 21 and the first-stage gear 13 rotate, and thereby the intermediate gear 15, the final-stage gear 17, and the advance screw 19 fixed to the final-stage gear 17 rotate.

The plunger 103 moves away from the final-stage gear 17 by the rotation of the advance screw 19 to squeeze out the liquid-drug in the syringe 101 through the tube 107 to the outside of the device. The back pressure on the plunger 103 is applied to the slit disk 23 via the advance screw 19, the final-stage gear 17, the intermediate gear 15, and the first-stage gear 13.

Under a normal state where the liquid-drug is normally squeezed out from the syringe 101, the back pressure on the slit disk 23 is constant. Thus, the fixed slit disk 21 and the slit disk 23 are kept in the initial state, and resilience of the torsion spring 25 causes the slit disk 23 to rotate in synchronization with the fixed slit disk 21.

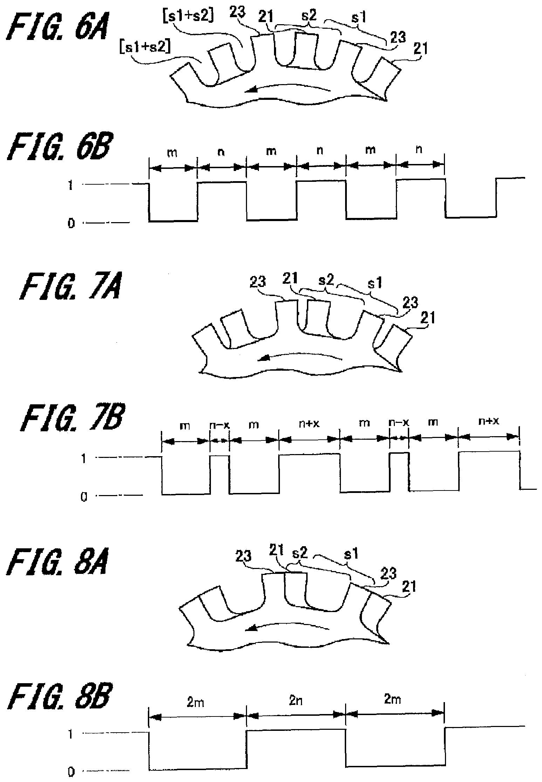

Under this state as illustrated in Fig. 6A, the fixed slit disk 21 and the slit disk 23 rotate, for example, with the slits s1 positioned to be shifted from the slits s2 by half a pitch. Therefore, as illustrated in Fig. 6B, the optical sensor 27 detects digital signals and counts detection signals "1" continuously n times in the overlapping region [s1 + s2] where the slit s1 overlaps the slit s2. Between the continuous counting of the detection signals "1" and the next continuous counting of the detection signals "1", detection signals "0" are continuously counted m times. Such is as described above.

Meanwhile, when the occlusion occurs in the passage for liquid-drug squeezed out from the syringe 101 by some cause, the back pressure applied to the slit disk 23 via the plunger 103 rises. Due to this rise in the back pressure, the torsion spring 25 for assembling together the slit disk 23 and the fixed slit disk 21 flexes by a degree corresponding to the rise in the back pressure. This restricts the rise in the back pressure being directly transmitted to the fixed slit disk 21.

Under this state, as illustrated in Fig. 7A, the rotation of the slit disk 23 is delayed from the rotation of the fixed slit disk 21 by a degree corresponding to the flex angle of the torsion spring 25. Therefore, as illustrated in Fig. 7B, the optical sensor 27 detects digital signals and counts detection signals "1" in the overlapping region [s1 + s2] where the slit s1 overlaps the slit s2. The number of continuously counted detection signals is n -x or n+ x, which alternately changes. Between the continuous counting of the detection signals "1" and the next continuous counting of the detection signals "1", detection signals "0" are continuously counted m times. Note that, x = 1 to n.

Worsening of occlusion in the passage for liquid-drug causes the rise in the back pressure applied to the slit disk 23, and thereby the flex angle of the torsion spring 25 increases. As illustrated in Fig. 8A, the rotation of the slit disk 23 is further delayed from the rotation of the fixed slit disk 21. Therefore, as illustrated in Fig. 8B, the detection signals "1" are continuously counted 2n times in the overlapping region [s1 + s2] where the slit s1 overlaps the slit s2, and the detection signals "0" are continuously counted 2m times between the continuous counting of the detection signals "1" and the next continuous counting of the detection signals "1".

Further worsening of occlusion in the passage for liquid-drug causes the slit s1 of the fixed slit disk 21 to completely match the slit s2 of the slit disk 23. Under this state, occlusion is most notably detected through analyzing of output signals. So the torsion spring 25 that reaches the maximum flex angle at this state may be used.

### Method of Occlusion Detection

For the liquid-drug administration device 1 operated as described above, an example algorithm for determining whether occlusion has occurred in the passage performed by the processing unit 29 is as follows.

According to signal data output from the optical sensor 27 as described with reference to Figs. 6A to 8B, the processing unit 29 determines the state as occlusion, for example, when continuous 2n-time counting of the detection signals "1" and continuous 2m-time counting of the detection signals "0" alternately happen. A state is determined as an occlusion state not only when the passage is completely occluded but also when the flow rate has decreased to a predetermined rate.

The light-emitting element constituting the optical sensor 27 is not necessarily of a pulse-transmitting type. For a light-emitting element other than a pulse-transmitting type, occlusion may be determined by the number of times of switching of output from the light-emitting element or the change in timing of switching. For example, in an occlusion state illustrated in Figs. 8A and 8B, the number of times of switching of the output from the light-emitting element in a predetermined time period is half the number of the normal state illustrated in Figs. 6A and 6B. The state may be determined as an occlusion state according to this change in the number of times of switching.

If the occlusion state determined by any of the methods described above has continued for a certain state, a signal that notifies that the state is an occlusion state is sent to the alarm unit 33 and then the alarm unit 33 sets forth an alarm. For a case where the liquid-drug administration device 1 is an insulin pump, for example, a signal is sent to the alarm unit 33 when an occlusion state has continued until the shortfall in the supplied amount of insulin liquid-drug reaches a predetermined value (for example, three units).

### Effect

In the liquid-drug administration device 1 configured as described above, occlusion detection is performed by an encoder that detects by the optical sensor 27 the difference in rotation between the slit disk 23 rotating in synchronization with the advance screw 19 and the fixed slit disk 21 fixed to the motor 11. This configuration enables performing occlusion detection with high accuracy compared to occlusion detection performed by an encoder using only a single slit disk.

Since the highly accurate occlusion detection is performed without combining occlusion detection that uses a diaphragm, the device can be downsized.

Furthermore, the occlusion detector 20 detects occlusion by only using the encoder and therefore is less exposed to liquid-drug than an occlusion detector 20 using a diaphragm. This allows the liquid-drug administration device 1 composed of disposable parts and reusable parts to include the occlusion detector 20 as a reusable part, which reduces cost of the device.

Furthermore, the fixed slit disk 21 and the slit disk 23 assembled together via the torsion spring 25 constitute a torque limiter. This configuration restricts the rise in the back pressure on the slit disk 23 caused by occlusion in the flow passage to be directly transmitted to the motor 11, which improves reliability of the device.

### Exemplary Modification

The present invention is not limited to the embodiment described above and illustrated in the drawings. Various modifications can be made without departing from the spirit and scope of the present invention according to the claims.

In the embodiment described above, for example, the occlusion detector 20 including the overlapping slit disk 23 and fixed slit disk 21 is provided between the motor 11 and the first-stage gear 13. A similar effect however can be obtained with any liquid-drug administration device according to the present invention provided with the slit disk 23 coaxially overlapping the fixed slit disk 21 via the torsion spring 25, where the fixed slit disk 21 rotates in synchronization with the motor 11 and the slit disk 23 rotates in synchronization with the advance screw 19.

Therefore, the occlusion detector 20 including the slit disk 23 overlapping the fixed slit disk 21 may be provided between the final-stage gear 17 and the advance screw 19. The occlusion detector 20 including the slit disk 23 overlapping the fixed slit disk 21 may be provided between two overlapping intermediate gears 15.

Furthermore, in the embodiment described above, the occlusion detector 20 including the overlapping single slit disk 23 and single fixed slit disk 21 is provided between the motor 11 and the first-stage gear 13. In the liquid-drug administration device according to the present invention, a plurality of slit disks 23 may be provided in a manner overlapping the fixed slit disk 21. In such a case, an elastic member, such as the torsion spring 25, is provided between each gap between the slit disks 23. In this manner, occlusion in a liquid-drug flow passage can be detected based on the change in the overlapping state of a plurality of slits.

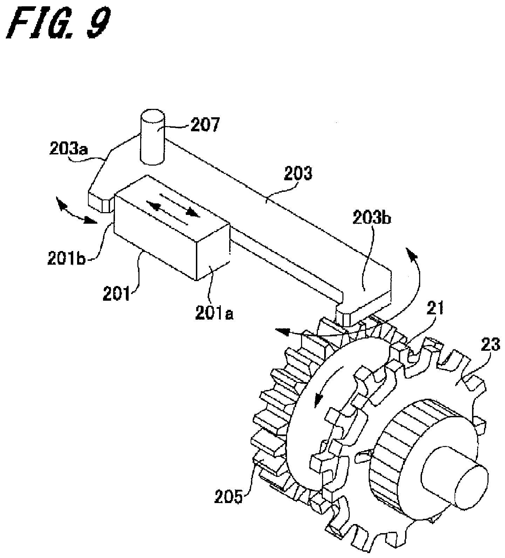

In the embodiment described above, the motor 11 is explained as an example actuator. The actuator however is not limited to that of a rotating type. For example, as illustrated in Fig. 9, an actuator using a piezoelectric element 201 to output rotational motion from linear motion may be used. When using the piezoelectric element 201, the piezoelectric element 201, a pivoting member 203 driven by the piezoelectric element 201, and a gear 205 driven by the pivoting member 203 are provided in the back side of the fixed slit disk 21.

The piezoelectric element 201 is positioned such that its one end that expands or contracts by an impressed voltage serves as a fixed end 201a and the other end serves as a moving end 201b.

The pivoting member 203 is a member extending along the expanding and contracting direction of the piezoelectric element 201. The pivoting member 203 is pivotally pinned at a shaft portion 207 provided vertical to the expanding and contracting direction of the piezoelectric element 201. The pivoting member 203 has on one end a force receiving end 203a that is pushed by the moving end 201b of the piezoelectric element 201. A loading end 203b that pushes the gear 205 in one direction is provided at the other end further away from the force receiving end 203a than the portion pinned by the shaft portion 207. By expansion of the moving end 201b of the piezoelectric element 201, the force receiving end 203a is pushed to pivot the pivoting member 203 about the shaft portion 207 and thereby the loading end 203b pushes the gear 205 to one direction.

The force receiving end 203a of the pivoting member 203 is coupled to the moving end 201b of the piezoelectric element 201 so as to follow the moving end 201b of the piezoelectric element 201. When the moving end 201b of the piezoelectric element 201 contracts and the force receiving end 203a follows the moving end 201b, the loading end 203b of the pivoting member 203 returns to its initial position without meshing with the gear 205.

As described above, the gear 205 driven by the piezoelectric element 201 and the pivoting member 203 is coaxially fixed to the fixed slit disk 21 described above.

The actuator described above controls the reciprocating motion of the loading end 203b and the rotational speed of the gear 205 by repetitive driving of the piezoelectric element 201.

The actuator thus configured can also be used like the motor 11 explained as the actuator in the embodiment described above.

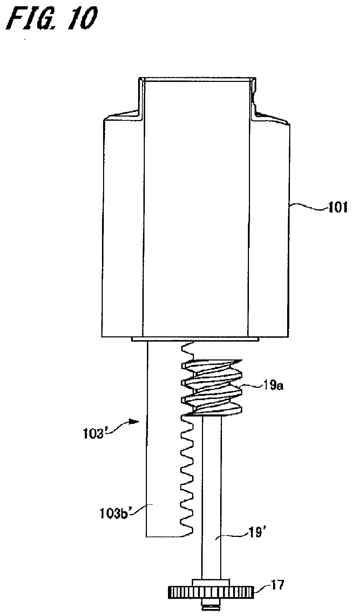

In the embodiment described above, an example where the advance screw 19 screwed in the nut 105 is provided as the output shaft for sliding the plunger 103 inside the syringe 101 is explained. The output shaft however is not necessarily provided as in the above configuration. For example, as illustrated in Fig. 10, a worm gear may be used.

In this case, a worm gear 19a is provided on an output shaft 19' that is provided upright on the final-stage gear 17 to be on the rotation axis of the final-stage gear 17. A shaft 103b' of the plunger 103' has throughout its longitudinal length a saw-tooth rack that meshes with the worm gear 19a. With such a configuration, the plunger 103' and the syringe 101, for example, can be provided as disposal parts detachable from the worm gear 19a serving as the output shaft.

### Reference Signs List

- 1: liquid-drug administration device
- 11: motor (actuator)
- 13: first-stage gear
- 17: final-stage gear
- 19: advance screw (output shaft)
- 19': worm gear (output shaft)
- 20: occlusion detector
- 21: fixed slit disk
- 23: slit disk
- 25: torsion spring (elastic member)
- 27: optical sensor
- 29: processing unit
- 101: syringe (liquid-drug container)
- 103, 103': plunger
- 105: nut
- 201: piezoelectric element (actuator)
- 203: pivoting member (actuator)
- 205: gear (actuator)
- s1: slit (fixed slit disk)
- s2: slit (slit disk)

## Claims

1. A liquid-drug administration device (1) comprising:
an actuator (11, 201, 203, 205);
a gear (13, 17) driven by the actuator (11, 201, 203, 205);
an output shaft (19, 19') provided on a rotation axis of the gear (13, 17) to slide a plunger (103, 103') in a liquid-drug container (101) containing liquid-drug; and
an occlusion detector (20) provided between the actuator (11, 201, 203, 205) and the output shaft (19, 19') to detect occlusion in a flow passage for liquid-drug supplied from the liquid-drug container (101), **characterized in that** the occlusion detector (20) includes a fixed slit disk (21) that has a slit (s1) in a disk face and
rotates in synchronization with rotation of an actuator (11) output, a second slit disk (23) that has a slit (s2) in a disk face and rotates in synchronization with rotation of the output shaft (19, 19'), the second slit disk (23) being coaxially and overlappingly assembled to the fixed slit disk (21), and an elastic member (25) that connects the fixed slit disk (21) to the second slit disk (23) to cause a delay in rotation of the second slit disk (23) with respect to the rotation of the fixed slit disk (21) in response to rise in back pressure on the second slit disk (23), whereby the occlusion detector (20) includes an optical sensor (27) that detects overlapping of the slit (s1) of the fixed slit disk (21) and the slit (s2) of the second slit disk (23), and a processing unit (29) that determines whether occlusion has occurred in a flow passage for liquid-drug supplied by driving of the output shaft (19, 19'), the determination being made based on a signal obtained by the optical sensor.

2. The liquid-drug administration device (1) according to claim 1, wherein
the elastic member (25) is a torsion spring, one end of the elastic member (25) being fixed to the fixed slit disk (21), another end of the elastic member (25) being secured on the second slit disk (23).

3. The liquid-drug administration device (1) according to any one of claims 1 to 2, further comprising:
a first-stage gear (13) as the gear provided on a rotation shaft of the actuator (11, 201, 203, 205); and
a final-stage gear (17) provided as the gear to mesh with the first-stage gear (13), wherein the output shaft (19, 19') is fixed on a rotation axis of the final-stage gear (17),
the fixed slit disk (21) is fixed to the rotation shaft of the actuator (11, 201, 203, 205); and
the second slit disk (23) is fixed to the first-stage gear (13).

4. The liquid-drug administration device (1) according to any one of claims 1 to 3, further comprising:
a liquid-drug container (101) having a supply port for liquid-drug;
a plunger (103, 103') inserted in the liquid-drug container (101); and
a nut (105) fixed to the plunger (103, 103') and screwed on the output shaft (19, 19'), the liquid-drug container (101), the plunger (103, 103'), and the nut (105) being provided on an axis of the output shaft (19, 19').

5. The liquid-drug administration device (1) according to claim 4, wherein
the liquid-drug container (101) and the plunger (103, 103') are detachable from a liquid-drug administration device (1) body including the occlusion detector (20).

## Patentansprüche

1. Flüssigarzneimittelverabreichungsvorrichtung (1), umfassend:
einen Aktuator (11, 201, 203, 205);
ein Zahnrad (13, 17), das durch den Aktuator (11, 201, 203, 205) angetrieben wird;
eine Abtriebswelle (19, 19'), die auf einer Drehachse des Zahnrades (13, 17) vorgesehen ist, um einen Kolben (103, 103') in einem Flüssigarzneimittelbehälter (101), der ein Flüssigarzneimittel enthält, zu verschieben; und
einen Okklusionsdetektor (20), der zwischen dem Aktuator (11, 201, 203, 205) und der Abtriebswelle (19, 19') vorgesehen ist, um eine Okklusion in einem Strömungskanal für ein aus dem Flüssigarzneimittelbehälter (101) zugeführtes Flüssigarzneimittel zu erfassen,
**dadurch gekennzeichnet, dass**
der Okklusionsdetektor (20) eine feststehende Schlitzscheibe (21) aufweist, die einen Schlitz (s1) in einer Scheibenfläche besitzt und sich synchron mit der Drehung eines Ausgangs des Aktuators (11) dreht, eine zweite Schlitzscheibe (23), die einen Schlitz (s2) in einer Scheibenfläche besitzt und sich synchron mit der Drehung der Abtriebswelle (19, 19') dreht, wobei die zweite Schlitzscheibe (23) koaxial zu der feststehenden Schlitzscheibe (21) ist und diese überlappend montiert ist, und ein elastisches Element (25), das die feststehende Schlitzscheibe (21) mit der zweiten Schlitzscheibe (23) verbindet, um in Reaktion auf einen Anstieg im Gegendruck auf der zweiten Schlitzscheibe (23) eine Verzögerung bei der Drehung der zweiten Schlitzscheibe (23) gegenüber der Drehung der feststehenden Schlitzscheibe (21) zu verursachen, wobei der Okklusionsdetektor (20) einen optischen Sensor (27) aufweist, der eine Überlappung des Schlitzes (s1) der feststehenden Schlitzscheibe (21) und des Schlitzes (s2) der zweiten Schlitzscheibe (23) erfasst, sowie eine Verarbeitungseinheit (29), die feststellt, ob es in einem Strömungskanal für ein durch den Antrieb der Abtriebswelle (19, 19') zugeführtes Flüssigarzneimittel zu einer Okklusion gekommen ist, wobei die Ermittlung anhand eines von dem optischen Sensor erhaltenen Signals erfolgt.

2. Flüssigarzneimittelverabreichungsvorrichtung (1) nach Anspruch 1, wobei das elastische Element (25) eine Torsionsfeder ist, wobei ein Ende des elastischen Elements (25) an der feststehenden Schlitzscheibe (21) befestigt ist und das andere Ende des elastischen Elements (25) an der zweiten Schlitzscheibe (23) befestigt ist.

3. Flüssigarzneimittelverabreichungsvorrichtung (1) nach einem der Ansprüche 1 bis 2, ferner umfassend:
ein Zahnrad (13) der ersten Getriebestufe als das auf einer Drehwelle des Aktuators (11, 201, 203, 205) vorgesehene Zahnrad; und
ein Zahnrad (17) der letzten Getriebestufe, das als das mit dem Zahnrad (13) der ersten Getriebestufe in Eingriff zu bringendes Zahnrad vorgesehen ist, wobei die Abtriebswelle (19, 19') auf einer Drehachse des Zahnrads (17) der letzten Getriebestufe befestigt ist,
die feststehende Schlitzscheibe (21) an der Drehwelle des Aktuators (11, 201, 203, 205) befestigt ist; und
die zweite Schlitzscheibe (23) an dem Zahnrad (13) der ersten Getriebestufe befestigt ist.

4. Flüssigarzneimittelverabreichungsvorrichtung (1) nach einem der Ansprüche 1 bis 3, ferner umfassend:
einen Flüssigarzneimittelbehälter (101) mit einer Zuführöffnung für Flüssigarzneimittel;
einen Kolben (103, 103'), der in den Flüssigarzneimittelbehälter (101) eingesetzt ist; und
eine Mutter (105), die an dem Kolben (103, 103') befestigt ist und auf die Abtriebswelle (19, 19') aufgeschraubt ist, wobei der Flüssigarzneimittelbehälter (101), der Kolben (103, 103') und die Mutter (105) auf einer Achse der Abtriebswelle (19, 19') vorgesehen sind.

5. Flüssigarzneimittelverabreichungsvorrichtung (1) nach Anspruch 4, wobei der Flüssigarzneimittelbehälter (101) und der Kolben (103, 103') von einem Körper der Flüssigarzneimittelverabreichungsvorrichtung (1), der den Okklusionsdetektor (20) aufweist, abnehmbar sind.

## Revendications

1. Dispositif d'administration de médicament liquide (1), comprenant :
un actionneur (11, 201, 203, 205) ;
un engrenage (13, 17), entraîné par l'actionneur (11, 201, 203, 205) ;
un arbre de sortie (19, 19'), prévu sur un axe de rotation de l'engrenage (13, 17), pour glisser un piston (103, 103') dans un conteneur de médicament liquide (101), contenant un médicament liquide et
un détecteur d'occlusion (20), prévu entre l'actionneur (11, 201, 203, 205) et l'arbre de sortie (19, 19'), pour détecter l'occlusion dans un passage d'écoulement pour le médicament liquide, fourni à partir du conteneur de médicament liquide (101),
**caractérisé en ce que**
le détecteur d'occlusion (20) comporte un disque fendu fixe (21), qui a une fente (s1) dans une face du disque et tourne en synchronisation avec la rotation de la sortie d'un actionneur (11), un second disque fendu (23), qui a une fente (s2) dans une face du disque et tourne en synchronisation avec la rotation de l'arbre de sortie (19, 19'), le second disque fendu (23) étant assemblé de manière coaxiale et chevauchante par rapport au disque fendu fixe (21) et à un élément élastique (25), qui relie le disque fendu fixe (21) au second disque fendu (23), pour causer un retard dans la rotation du second disque fendu (23) par rapport à la rotation du disque fendu fixe (21), en réponse à l'augmentation de la contre-pression sur le second disque fendu (23), le détecteur d'occlusion (20) comportant un capteur optique (27), qui détecte le chevauchement de la fente (s1) du disque fendu fixe (21) et de la fente (s2) du second disque fendu (23) et une unité de traitement (29), qui détermine si l'occlusion s'est produite dans un passage d'écoulement pour le médicament liquide, fourni par l'entraînement de l'arbre de sortie (19, 19'), la détermination étant faite en fonction d'un signal, obtenu par le capteur optique.

2. Dispositif d'administration de médicament liquide (1) selon la revendication 1, dans lequel
l'élément élastique (25) est un ressort de torsion, une extrémité de l'élément élastique (25) étant fixée au disque fendu fixe (21), une autre extrémité de l'élément élastique (25) étant fixée sur le second disque fendu (23).

3. Dispositif d'administration de médicament liquide (1) selon l'une quelconque des revendications 1 à 2, comprenant en outre :
un premier étage d'engrenage (13), en tant qu'engrenage, prévu sur un arbre de rotation de l'actionneur (11, 201, 203, 205) et
un étage final d'engrenage (17), prévu en tant qu'engrenage, pour s'engrener avec le premier étage d'engrenage (13), dans lequel l'arbre de sortie (19, 19') est fixé sur un axe de rotation de l'étage final d'engrenage (17), le disque fendu fixe (21) est fixé à l'arbre de rotation de l'actionneur (11, 201, 203, 205) et le second disque fendu (23) est fixé au premier étage d'engrenage (13).

4. Dispositif d'administration de médicament liquide (1) selon l'une quelconque des revendications 1 à 3, comprenant en outre :
un conteneur de médicament liquide (101), ayant un orifice d'alimentation pour le médicament liquide ;
un piston (103, 103'), inséré dans le conteneur de médicament liquide (101) et
un écrou (105), fixé au piston (103, 103') et vissé à l'arbre de sortie (19, 19') le conteneur de médicament liquide (101), le piston (103, 103') et l'écrou (105) étant prévus sur un axe de l'arbre de sortie (19, 19').

5. Dispositif d'administration de médicament liquide (1) selon la revendication 4, dans lequel le conteneur de médicament liquide (101) et le piston (103, 103') peuvent être détachés d'un corps du dispositif d'administration de médicament liquide (1), comportant le détecteur d'occlusion (20).
